# EUROPEAN PATENT SPECIFICATION

(11) **EP 2 563 220 B1**
(45) Date of publication and mention of the grant of the patent: **23.07.2014**
(21) Application number: 11719608.9
(22) Date of filing: 21.04.2011
(51) Int. Cl.: A61B 5/107, A61B 5/00, A61B 5/103, A61Q 19/00

(54) **SKIN PROFILING AND ITS USE IN TESTING COSMETICS**
HAUTPROFILIERUNG UND DEREN VERWENDUNG BEIM TESTEN VON KOSMETIKA
ÉTABLISSEMENT DE PROFILS DE PEAU ET SON UTILISATION DANS L'ESSAI DE PRODUITS COSMÉTIQUES

(30) Priority: 18.11.2010 GB 201019523; 26.04.2010 GB 201006953
(43) Date of publication of application: 06.03.2013
(73) Proprietor: AME Health Ltd, Croydon, Surrey CR0 8UJ (GB)
(72) Inventor: BARKER, Stephen, George, Edward, Surrey CR0 8UJ (GB); HOPPER, Colin, Greater London NW1 2PG (GB)
(74) Representative: Stevens, Fiona
(86) International application number: PCT/GB2011/050802
(87) International publication number: WO 2011/135341

(56) References cited:
- EP-A1- 1 920 714
- WO-A2-2007/026340
- WO-A2-2007/113758
- US-A1- 2001 053 347
- US-A1- 2005 251 049

## Description

### Field of the Invention

This invention relates to skin testing and, more particularly, to a method of testing the effectiveness of cosmetics.

### Background of the Invention

Women in particular, although not exclusively, are concerned about the appearance of wrinkles and the "plumpness" of their skin. Plumpness is essentially a reflection of the degree of hydration of the skin. The sales of anti-wrinkle formulations and "hydrating" agents are high.

Anti-wrinkle preparations work principally, by filling in wrinkles on the skin surface. Hydrating agents can act either to prevent moisture loss from the skin, or by increasing moisture passing in to the skin layers. M easuring the effectiveness of such preparations is neither quick nor very practical. At present, it can only be done effectively by animal testing, requiring both sensitisation studies and eventual sacrifice of the test animals. This testing can be counterproductive for companies that wish to promote the general acceptability of such formulations.

Optical Coherence Tomography (OCT) is an imaging technique, normally based around an interferometer which is fed by a broadband light source to produce a two-dimensional image from internal tissue microstructure in a way that is analogous to confocal microscopy or ultrasound. OCT generally uses wavelengths within the bandwidth 600-2000 nm, where the main constituents of the tissue, water and pigments exhibit low absorption. Beam splitters divide the light passing through the interferometer into two different paths: the "reference" and the "sample" arms. The reflected beams from a reference mirror and from a scattering sample then pass back to the interferometer and generate a cross-correlation signal which is directed towards a detector. The detection of an interferometric signal is possible only when the sample and reference signals are almost matched in "time of flight".

By way of example, the VivoSight Multi-beam OCT scanner available from Michelson Diagnostics Limited, is capable of scanning the epidermis, dermis, hair follicles, etc. in 2-D and 3-D, and the skin layers, to a depth of 1 mm or more. Such a scanner is described in WO2006/054116 and WO2008/068497 . Such apparatus is typically used to determine the presence/spread of cancer cells beneath the skin surface. Another example is the device being developed by lmalux in the United States. Other imaging modalities that might have a similar capacity to 'examine' the skin layers are in development too.

WO2008/112203 discloses a method for identifying a sensor site by obtaining local skin spectrum data. In one embodiment, the location, e.g. forehead or forefinger, of a spectrophotometer sensor, e.g. a pulse oximeter, is determined by measuring spectra indicating the water content in the epidermis and dermis, and their relative thicknesses.

US 2001/053347 discloses that retinol treated skin shows a thickened epidermis and an increased interface surface between dermis and epidermis by the presence of new rete pegs and papillae.

### Summary of the Invention

The present invention is based on the realisation that apparatus of the types d escribed above can be used to provide a non-invasive, quantitative measure (principally as one or more ratios) of the effectiveness of a cosmetic or "cosmeceutical", by comparing the relative thickness of the dermis and epidermis, and/or the changing or effect on pattern of the "rete ridges" as defining the boundary between the dermal and epidermal layers of the skin (principally as a change in defined length). More immediately, the present invention can be used to obtain an immediate measure of a subject's suitability for treatment with any given cosmetic, by observing changes in such thicknesses (i.e. a change in the ratio) and/or the rugosity of the rete ridges (i.e. a change in the defined length) or a derived volume of the epidermis relative to the dermis. It will be appreciated that, as a cosmetic may act to promote increases in the blood supply to a given region, and thereby act to increase the apparent plumpness of the skin (through an increase of the water content locally of the skin), the thickness of the dermis will increase relative to the epidermis, or vice versa, and the rete ridges will be likely to be flattened, so that their length with respect to a given length of skin surface will be reduced.

According to one aspect of the invention, a method of profiling skin, comprises comparing the rugosity and/or the length of rete ridges beneath the skin relative to a length of skin and comparing the thicknesses of the epidermis and dermis.

According to another aspect of the invention, a method for determining the effectiveness of a cosmetic preparation on skin, comprises the steps of claim 2.

### Description of the Invention

The example devices used to assess the skin as described above can also be used to determine the depth of fill in a wrinkle, if the agent that is used is of that type. In addition, they can be utilised to examine stretch marks, wound-healing, scar formation, tissue regeneration, e.g. after a burn, and more generally, skin conditions and/or pathological skin conditions.

The invention has been described above with particular reference to the dermis, epidermis and the rete ridges which, as is generally known, are undulations at the dermo-epidermal junction. These are only examples of skin parts which have reasonably well-defined boundaries and may be most likely to be measured and/or affected by treatment. For the purposes of the present invention, other layers and/or boundaries and their absolute or relative thicknesses may be considered instead. These include the stratum corneum, stratum lucidum, stratum basale, stratum spinosum and stratum granulosum; see, for example, Gray's Anatomy, 35th British Edition, Ed's: Warwick and Williams, published by W.B.Saunders Company, pages 1159-1169.

The skilled man will appreciate that each of the criteria may need to be determined as a mean (or average). Thus, for example, the thickness of the epidermis may be determined from the skin surface to the boundary of the epidermis and dermis, and that latter boundary may be an average calculated on the basis of the undulations of the rete ridges. The thickness of the dermis may be determined as a distance between an average of the rete ridges and an estimated/averaged position of the generally planar capillary plexus more towards the junction of the dermis and fat layer beneath the skin, e.g. with respect to points of blood flow.

As indicated above, but merely for the purposes of illustration, each of the parameters to which the present invention relates may be determined by OCT, e.g. using apparatus of the type described in WO2006/054116 and WO2008/068497 or that being developed by lmalux in the United States. Any other modality, however, could be used to measure skin thickness. Laser light is currently preferred, but it could be an ultrasound-based device, or MRI scan, or other wavelength-based system.

Where comparison of two measurements is required, for example to obtain the aforementioned ratio (expressed perhaps as "AME units" - advanced measurements of the epidermis), that can be done *ex vivo,* e.g. using a computer programmed accordingly, or undertaken by a manual means of calculation. When a single measurement is taken, it may be appropriate to store that information together with other characteristics that may be relevant in context, i.e. with respect to skin tone and/or turgor, and typically in association with a subject's other characteristics.

Each measurement that is made will typically be over a given area of skin, e.g. 5 mm x 5 mm. When measuring the rugosity of the rete ridges, or the flattening or increased undulation thereof, that will typically be done with respect to a given length, e.g. 5 mm, of overlying skin surface. Measurements may be taken in a more complex way, for example, calculated from assessments undertaken in three dimensions, i.e. from a volume assessment of a portion of skin.

The utility of the invention is illustrated by a study in which "normal" skin only, i.e. without any creams or lotions applied, is scanned. The scanned areas may be the back of the left hand, the front surface of the left forearm (the volar surface) and the highest point of the left cheek (on the face). A surface block of tissue is scanned, for example at 3 mm x 3 mm and to a depth of 1.5 mm. Scans are recorded and saved for later processing to give the measurements as described above. Details for each person are taken to include name, age, date of birth and notes, e g. white or black skin.

More specifically, in a study, data were collected using an available clinical OCT unit (Michelson Diagnostic VivoSight) featuring proprietary MultiBeam technology. Three different skin areas were profiled; the volar surface of the forearm, the back of the hand, and the highest point of the face on the cheek. All tests were done on the left side of the body for simple consistency. 50 volunteers in the age ranges of 16-25 years, 30-45 years and 55+ years were examined. In each case, skin type and colour were noted. Skin was not affected in any way by taking the measurements. All data were imported using appropriate software and, subsequently, the thicknesses of the epidermis and dermis were measured, together with a review of the skin surface. OCT works by detecting scattered infrared light and, typically, can see through 1-2 mm of "opaque" material and provide information that gives users the ability to examine structures which extend far below the surface. The Vivosight scanner provides high resolution, at better than 7.5 µm lateral and 10 µm vertical resolution.

Each examination took less than 20 seconds to complete per site. Volunteers were able to see their skin profile immediately. Measurements were possible for the epidermis and the dermis at the time of examination from the machine screen although, in this study, images were captured and data obtained at a later time. Measurements in this study were relatively simple as no mechanical (or other) interventions were used to alter structure in a test manner.

The OCT device combines the entire instrumentation necessary on one robust trolley that is ready to be used in a clinical environment. A hand-held probe (incorporating the lens of a Class 1 laser) is ergonomically designed and is easy to hold and use. A spacer component holds the lens away from the skin surface and is easy to clean. The system is robust enough that the probe can be held firmly on the skin and can be moved during an examination, with real-time images provided of the movement (and its effects on the dynamics of the skin). Image capture (to a contained hard drive within the system's computer) is rapid, taking a matter of seconds only. Examples of 2-D and 3-D images of the skin from individuals were easy to obtain and review, on computer disc or as hard copy.

Individuals scanned were sitting comfortably, and their personal demographics were recorded prior to examination. Examination was completely non-invasive, painless and was deemed safe; a precaution to close the eyes when assessing the skin over the face cheek was deemed prudent.

Skin anatomy was clear to see. The epidermis was well-defined, and wrinkles, hair follicles and hair were easily visualized together with pores and glands. The undulations of the epidermis:dermal junction were clear to see and the dermal layer was distinguishable. The capillary plexus deeper within the dermis was clearly detectable, though its exact deep boundary with the dermis was often difficult to identify. By bracketing the participants into age ranges (16-25 years, 30-45 years and 55+ years), an attempt was made to define differences in the skin anatomy, particularly between skin on the volar surface of the (left) forearm, the back of the (left) hand, and the highest point of the face (left cheek). Results indicated that skin on the volar surface of the forearm, in each age grouping, had a thicker epidermis than over the cheek, but thinner than on the back of the hand. The epidermal:dermal junction was most obvious over the cheek. The epidermis and the dermis in these were much thinner than at the other two sites.

The capillary plexus was most pronounced (i.e. with the greatest number of capillaries located) in the skin of the face. Importantly, no clear differences could be seen at the three sites examined, between white skin, black skin and skin from any other racial group. Surprisingly, there were seemingly insignificant differences between the skin anatomies of all but the very oldest subjects examined. For practical purposes, it was noted that review of the skin profile was in fact easier from the screen in real-time than when reviewing images captured on the device's associated hard drive; measurements of the epidermal and dermal thicknesses were in fact easier from the screen at the actual time of the study being performed.

In utilizing this new technology, it was found that OCT scanners allow an extremely easy demarcation of the skin surface, as the brightest white line on the scan. The undulations of the rete ridges are often clearly seen. For example, these were more obvious in the cheek than on the back of the hand. However, such undulations make the definition of a precise depth to the junction difficult to determine. An algorithm was created that averages the undulations (from the block of tissues assessed) to approximate a straight line. It is then possible to calculate the mean distance from the skin surface to the respective line, which represents a tissue layer. Because each pixel has a defined resolution, the distance between tissue surface and layer gives a straightforward measure of the thickness of a layer, denoted as "x".

The least deep part of the dermal layer (i.e. at the epidermal:dermal junction) is most often obvious, and the deeper capillary plexus can often be clearly seen. However, the deep boundary of the dermis *per se* was often hard to define. For the purposes of this study, the averaged depth to the position of the top of capillary plexus is taken as the *de facto* boundary of the dermal layer; the capillary plexus undulates in 3-D, but lies in an essentially 2-D plane. As the OCT scanner scans a block of tissue, so it becomes possible to reconstruct a 3-D image of that block. Software allows the multiple points measured down from the epidermal:dermal junction to the top of capillary plexus. This provides the "y" measurement required.

It was concluded that the ratio of the epidermal:dermal ("x/y") thickness is one of the most useful measures identified herein. This ratio has been expressed as epidermis over dermis, to give a ratio ("AME ratio", herein), typically in the skin of the volar surface of the forearm, back of the hand and in the cheek (see drawings). Further, the undulations of the rete ridges present a sinusoidal boundary layer whose length ("z") can be measured against a fixed length across the surface of the skin, e.g. per linear mm. A ratio can now be created that compares the length of the boundary to a fixed five mm length on the skin surface, expressed as "z"/5 mm and referred to as the "rete ratio". Between them, these two ratios provide the basis for a standard to define just how much any applied cosmetic or cosmaceutical agent affects the volume, "hydration" or "plumpness" of the skin.

The ability of OCT to scan the skin surface allows, in addition, an estimate of "depth of wrinkle fill" by so-called anti-wrinkle agents. For compounds such as depilatories (or devices such as razors), it can provide an accurate assessment of their function. In the medical field, similar examinations could be made to determine effectiveness of medicines, e.g. the rate/extent to which psoriatic plaque is thinned and reduced in size by topical application of a specific trial agent.

The usefulness of these ratios is evident in the assessment of an individual's skin in a "resting" or non-pathological state. An AME ratio and a rete ratio can be quoted for any target area of skin examined. A subsequent assessment of the same area of skin, whether affected by a pathological condition, or altered from its "resting" state by the application of a particular agent, might be expected to alter these key ratios. For example, onset of psoriasis, with formation of thicker plaques or principally epidermal skin, would be expected to substantially diminish the AME ratio (as the epidermal layer thickens in relation to the dermal layer); with appropriate treatment, the original AME ratio might be obtained again. As another example, application of various cosmetic agents might bulk either the epidermal or dermal layers, altering the AME ratio or, perhaps, cause the epidermal:dermal junction to stretch and hence, shorten, per unit linear skin surface, giving a reduction in the rete ratio. It will be appreciated that, by reviewing these two ratios, the effectiveness, or lack of, of any given agent purporting to affect the skin, could be assessed and compared to others in turn.

"Skin profiling" could become the best way to truly define an individual's surface anatomy, and the anatomy of the deeper layers, allowing the best available cosmetics or cosmaceuticals to be recommended and applied to enhance the final appearance of the skin. Anti-wrinkle creams, for example, could be seen in real-time for their ability to "in-fill" wrinkles but importantly, the depth of in-fill, or the sustainability of in-fill. The use of moisturising formulations (skin-plumping agents) might be seen in real-time also, for their effect to enhance volume of the epidermal or dermal layers, and to smooth out the undulations of the rete ridges, manifest on the surface perhaps. By the same logic, OCT skin profiling may find security applications in rapid validation of individual identity, for example at airports.

## Claims

1. A method of profiling skin which comprises comparing the rugosity and/or the length of rete ridges beneath the skin relative to a length of skin, and additionally comparing the thicknesses of the epidermis and the dermis.

2. A method for determining the effectiveness of a cosmetic preparation on skin, which comprises determining the rugosity and/or length of rete ridges before and after treatment with the cosmetic preparation, wherein reduced rugosity and/or reduced length of the rete ridges is indicative of the ability of the cosmetic preparation to hydrate the skin, and additionally comparing the thickness of the epidermis and the dermis before and after treatment with the cosmetic preparation, wherein an increased thickness of the dermis relative to the epidermis, or *vice versa,* after treatment is indicative of the ability of the cosmetic preparation to hydrate the skin.

## Patentansprüche

1. Verfahren zur Profilierung der Haut, die den Vergleich der Rautiefe und/oder der Länge der Malpighi-Erhöhungen unter der Haut relativ zu einer Länge von Haut umfasst, und zusätzlich die Dicke der Epidermis und der Dermis vergleicht.

2. Verfahren zur Bestimmung der Wirksamkeit eines kosmetischen Präparats auf der Haut, die die Bestimmung der Rautiefen und/oder der Längen von Malpighi-Erhöhungen vor und nach der Behandlung mit dem kosmetischen Präparat umfasst, wobei eine reduzierte Rautiefe und/oder eine reduzierte Länge der Malpighi-Erhöhungen für die Fähigkeit des kosmetischen Präparats, die Haut zu hydratisieren, indikativ ist, und zusätzlich die Dicke der Epidermis und der Dermis vor und nach der Behandlung mit dem kosmetischen Präparat vergleicht, wobei eine erhöhte Dicke der Dermis relativ zur Epidermis, oder umgekehrt, nach der Behandlung für die Fähigkeit der kosmetischen Präparation, die Haut zu hydratisieren, indikativ ist.

## Revendications

1. Méthode de profilage de la peau qui comprend les étapes consistant à comparer la rugosité et/ou la longueur des crêtes réticulées sous la peau à une longueur de peau et, en outre, à comparer les épaisseurs de l'épiderme et du derme.

2. Méthode de détermination de l'efficacité d'une préparation cosmétique sur la peau, qui comprend les étapes consistant à déterminer la rugosité et/ou la longueur des crêtes réticulées avant et après le traitement avec la préparation cosmétique, dans laquelle une rugosité réduite et/ou une longueur réduite des crêtes réticulées est indicative de l'aptitude de la préparation cosmétique à hydrater la peau et, en outre, à comparer l'épaisseur de l'épiderme et du derme avant et après le traitement avec la préparation cosmétique, dans laquelle une augmentation de l'épaisseur du derme par rapport à celle de l'épiderme, ou *vice versa,* après le traitement est indicative de l'aptitude de la préparation cosmétique à hydrater la peau.
